# EUROPÄISCHE PATENTSCHRIFT

(11) **EP 1 303 297 B1**
(45) Veröffentlichungstag und Bekanntmachung des Hinweises auf die Patenterteilung: **17.11.2004**
(21) Anmeldenummer: 01951556.8
(22) Anmeldetag: 02.06.2001
(51) Int. Cl.: A61K 38/42, A61K 47/32

(54) **SAUERSTOFFTRÄGER ENTHALTENDE ZUBEREITUNG ZUR REGENERATION DER HAUT BEI SAUERSTOFF-MANGEL**
PREPARATION CONTAINING AN OXYGEN CARRIER FOR REGENERATION OF THE SKIN IN THE CASE OF OXYGEN DEFICIENCY
PREPARATION CONTENANT UN PORTEUR D'OXYGENE POUR LA REGENERATION DE LA PEAU EN CAS D'INSUFFISANCE D'OXYGENE

(30) Priorität: 29.06.2000 DE 10031741
(43) Veröffentlichungstag der Anmeldung: 23.04.2003
(73) Patentinhaber: SanguiBioTech GmbH, 58455 Witten (DE)
(72) Erfinder: BARNIKOL, Wolfgang, D-55128 Mainz (DE)
(74) Vertreter: Müller, Claudia Dr.
(86) Internationale Anmeldenummer: PCT/EP2001/006328
(87) Internationale Veröffentlichungsnummer: WO 2002/000246

(56) Entgegenhaltungen:
- EP-A- 0 673 643
- WO-A-98/06438
- FR-A- 2 641 463
- FR-A- 2 741 266

## Beschreibung

Die Erfindung betrifft ein extern applizierbares Sauerstoffträger-haltiges Präparat, wobei Hämoglobin und gegebenenfalls Myoglobin in einer Zubereitung gelartiger Konsistenz molekular-dispers eingearbeitet ist. Das Präparat eignet sich zum Einreiben in die Haut, um die diffusive Versorgung der Haut mit Sauerstoff von außen zu ihrer Regeneration und Behebung des Sauerstoffmangels zu verstärken. Das Mittel eignet sich auch zur Prävention solcher Zustände, und ist besonders geeignet bei degenerativen, strahlungs-, thermisch- und altersbedingten Hautveränderungen, auch nach Hautverbrennungen, gegebenenfalls in Co-Therapie mit intravasaler Sauerstofftherapie.
Die Erfindung betrifft ferner ein Verfahren zur Herstellung eines solches Präparates und dessen Verwendung.
Eine Reihe degenerativer Veränderungen der Haut werden durch chronischen Sauerstoffmangel hervorgerufen. Ein solcher Mangel entsteht dann, wenn die Haut nicht mehr ausreichend durchblutet ist. Dies geschieht entweder bei einer Einengung der kleinen Arterien - der Blut- Zufuhrgefäße - oder aber im Falle eines venösen Staus - Venen sind die Abflußgefäße des Organismus; betroffen sind vorzugsweise die Beine.
Der Kliniker kennt hierzu ganz bestimmte Krankheitsbilder, beispielsweise die chronische periphere Verschlußkrankheit mit ihren vier verschiedenen Stadien nach Fontaine oder die diabetische Angiopathie, deren Ursache eine Arteriosklerose ist, oder auch die chronische Veneninsuffizienz, d.h. eine Malfunktion von Gefäßen.
Der chronische Sauerstoffmangel führt schließlich zum geweblichen Zerfall der Haut, auch in Form der Gangrän oder des Ulcus cruris, sogen. offenes Bein. Ist die Sauerstoffversorgung grenzwertig, welches oft für alte Menschen zutrifft, so führen schon relativ kurzzeitige Kompressions-Anämien, wie sie während des festen Liegens vorkommen, oder nur geringfügiges Anstossen zu schnellem Zerfall zuerst der Haut und darauf auch zum Untergang der darunter liegenden Gewebe, was man als Dekubitus bezeichnet. Es wäre von Vorteil, hier vorbeugende Methoden anwenden zu können, um prophylaktisch die genannten krankhaften und schmerzhaften Zustände zu vermeiden.
Eine ausführliche Darlegung der dermatologischen Klinik hierzu findet sich bei Braun-Falco, "Dermatologie und Venerologie", Springer-Verlag, ISBN 3-540-53542-X.

Ein weiteres wichtiges dermatologisches Problemfeld sind Hautschäden nach Bestrahlungen. Man findet in diesem Fall entzündliche und degenerative Veränderungen. Es kann somit auch hier davon ausgegangen werden, dass eine verbesserte diffusive Sauerstoffversorgung der Haut von außen solche Schädigungen hintanhalten kann; möglich ist auch hierbei eine prophylaktische Therapie.
Ein drittes wichtiges Problemfeld sind Hautschäden nach Verbrennungen; auch hierbei kann eine verstärkte diffusive Sauerstoffversorgung von außen helfen, die Haut besser und schneller zu regenerieren.

Die sichtbare äußere Schicht der Haut besteht aus etwa 15 Zelllagen verhornender, d.h. absterbender, sehr flacher Zellen (Hornzellen), diese Schicht (Stratum corneum) ist an der normalen Haut etwa 12 µm dick, was in etwa dem Durchmesser rundlicher Körperzellen entspricht. Die Hornzellen schilfern ständig ab und entstehen durch Teilung im darunter befindlichen sogen. Stratum germinativum; darüber befindet sich das Stratum granulosum. Diese beiden vitalen Zellschichten der Epidermis sind zusammen etwa 25 µm dick. Damit bestehen für die Sauerstoffdiffusion, was die geometrischen Verhältnisse betrifft, innergewebliche Verhältnisse; denn der Versorgungsbereich einer Kapillare - das ist die kleinste Gefäßart des Organismus - besitzt eine Tiefe von rund 50 µm. Es dauert etwa 1 Monat bis eine Basalzelle des Stratum germinativum an der Oberfläche als Keratinozyt von der Hautoberfläche abgestoßen wird.
Unter der Epidermis liegt die Dermis: diese wölbt sich in Form vieler Papillen in die Epidermis hinein, und in jeder Papille befindet sich eine versorgende Kapillare mit ihrem arteriellen und venösen Ende. Von dieser Kapillare diffundiert der Sauerstoff auswärts zur unteren vitalen Schicht der Epidermis, dem genannten Stratum germinativum. Die Epidermis erhält nun den notwendigen Sauerstoff aber nicht nur von innen, sondern - wie gezeigt wurde (beispielsweise Großmann et al., Adv. Physiol. Sci. 25 (1981): Oxygen Transport to Tissue. 319-32p oder L.R. Fitzgerald, Physiol. Rev. 37 (1957) 325-336) - sie versorgt sich zu etwa 50% von außen diffusiv mit Sauerstoff.

Wenn nun, wie oben ausgeführt, von der vaskulären Seite her, durch krankhafte Gefäßveränderungen eine mangelhafte Sauerstoffversorgung vorliegt, so besteht das Problem und die Aufgabe, anderweitig für eine Kompensation zu sorgen.

FR-A-2 741 266 offenbart ein kosmetisches Gel mit Hämocyanin

Aufgabe vorliegender Erfindung ist daher die Entwicklung eines Mittels zur Steigerung der externen Sauerstoffversorgung der Haut, mit der die oben genannten Erkrankungen behandelt und verhindert werden können.

Diese Aufgabe wird erfindungsgemäß gelöst durch ein Präparat gemäss den Ansprüchen 1-7, dessen Herstellungs- verfahren gemäss den Ansprüchen 8-11 und dessen Verwendung gemäss den Ansprüchen 12-16. Insbesondere ist eine Lösung eines Sauerstoffträgers, bzw. Hämoglobin oder Hämoglobin und Myoglobin insbesondere in einer Wasser und ggf. Salze enthaltenden Lösung in eine Zubereitung gelartiger Konsistenz molekular-dispers eingearbeitet, wozu eine Gel-bildende Substanz (0,1-20, vorzugsweise 0,1-8%) vorhanden ist. Insbesondere ist die gesamte Lösung in ein Gel eingearbeitet, wie anorganische Gele (Bentonit, Kieselsäure) und auf organischer Grundlage, wie Polyacrylsäure, Gummiarabicum, Pectinalginate, Methylcellulose, Hydroethylcellulose, Stärke sowie Carboxymethylcellulose. Vorzugsweise ist das Gel ein Hydrogel, ausgewählt aus anionischen Polyacrylaten, insbesondere Carbopol® der verschiedenen Typen wie Carbopol 940 oder 940 P. Das Gel ist fettfrei.
Als Salze können natürliche Elektrolytkomponenten der Lösung des Hämoglobins/Myoglobins wie NaCl, KCl und NaHCO3, insbesondere in folgenden physiologischen Mengen vorliegen (in mM): NaCl 125; KCl 4,5; NaHCO₃ 2,0.

Das verwendete Gel enthält auch Konservierungsstoffe wie Dibromhexamidin, Dihydracetatsäure, 4-Hydroxybenzoesäure, Benzoesäure, Propionsäure, Salicylsäure, Sorbinsäure, Formaldehyd, Paraformaldehyd, O-Phenylphenol, anorganische Sulfite und Bisulfite, Natriumjodat, Chlorobutanol und Ameisensäure; in Fällen von Säuren kommen auch deren Ester und Salze in Frage. Die Mittel sind in Mengen von 0,15-0,25 oder wie unten geschildert vorhanden. Bevorzugt werden Konservierungsmittel ausgewählt aus Methyl-4-Hydroxy-Benzoat und Propyl-4-Hydroxy-Benzoat, z.B. 0,15-0,25, insbesondere 0,05-0,2, ganz besonders 0,09-0,17 %. Ferner sind Feuchthaltemittel wie Na-Lactat,Glycerol, Propylenglycol, Prbpandiol, Sorbit, PCA (Pyrrolidoncarbonsäure), in einer Menge von 5-15% enthalten Besonders bevorzugt sind Methyl-4-Hydroxy-Benzoat und Propyl-4-Hydroxy-Benzoat als Konservierungsmittel und Glycerol, Propylenglycol und Sorbit als Feuchthaltemittel, insbesondere jeweils Mischungen hiervon, z.B. 1:1 betreffend Konservierungsmittel und 1:1:1 bezüglich Feuchthaltemittel. Ein besonders bevorzugtes erfindungsgemässes Präparat gelartiger, Konsistenz umfasst Hämoglobin oder, Hämoglobin/Myoglobin ( 0,1-20%, bezogen auf die Gesamtmasse), 5-15 Gew.-% Feuchthaltemittel, 0,15-0,25 % Konservierungsmittel, und 0,1 bis 20 %, insbesondere 0,1 bis 8 % Carbopol. Ganz besonders bevorzugt sind Präparate auf Basis von 1-5% Carbopol®, 0,02-0,08 Propyl-4-Hydroxybenzoat, sowie 0,07-0,15 Masseteile Methyl-4-Hydroxybenzoat, 8-12% Glycerol, Propylenglycol und/oder Sorbit (1:1 oder 1:1:1). Bevorzugt liegt Hämoglobin oder Hämoglobin und Myoglobin in einer Menge von 2-8% vor. Die angegebenen Mengen sind jeweils Gewichtsprozent.
Das Hämoglobin kann insbesondere bevorzugt in der erfindungsgemässen Zubereitung ein mit Kohlenmonoxid ( CO ) stabilisiertes Hämoglobin, vorzugsweise Schweinehämoglobin ( 0,1-20%, insbesondere 2-8% ) sein. Die Herstellung eines solchen stabilisierten Hämoglobins ist in der DE 1 970 103.7 beschrieben. (entsprechend US-Patent Nr. 5,985,332). Demnach kann Hämoglobin/Myoglobin durch Aquilibrierung mit CO vollständig in Carboxyhämoglobin/Myoglobin überführt werden, welches lagerstabil ist und vor der weiteren intravalen Anwendung nicht deligandisiert werden muss. Die Karbonylierung ist auch mit modifiziertem Hämoglobin möglich.

Die Aktivierung des Sauerstoffträgers erfolgt dann durch Begasung der Haut mit Sauerstoff, auf die das Gel aufgetragen worden ist.

Das Hämoglobin kann, wie erwähnt, in einer Mischung mit Myoglobin, insbesondere letzteres in Mengen von 0,1 bis 50 Gew.-%, bezogen auf die Hämoglobin-Menge, vorliegen. Bevorzugt wird Myoglobin mit Hämoglobin in Mengen von 50 bis 70% Hämoglobin und 50-30% Myoglobin, insbesondere 75 bis 90% Hämoglobin und 25 bis 10%, Myoglobin eingesetzt. Die %-Angaben sind hierbei Masseanteile.

Das Hämoglobin oder Hämoglobin und Myoglobin liegen in bestimmten Konzentrationen, wie angegeben, in den erfinduhgsgemäß beschriebenen molekularen Formen in einer wasserbindenden und tiefenwirksamen gelartigen Zubereitung, z.B. in einem Gel, molekular-dispers vor.

Überraschenderweise ist erfindungsgemäß mit Hilfe derart eingearbeiteter Hämoglobine oder Hämoglobin und Myoglobin in Gel oder gelartige Lösungs-Strukturen, insbesondere von modifizierten und nicht modifizierten (nativen) molekular-dispersen Hämoglobinen bzw. Hämoglobinen und Myoglobin eine erleichterte Diffusion und damit erhöhter Sauerstofftransport von außen durch die Epidermis möglich. Vorteilhaft ist bei Einsatz nativer Hämoglobine, deren sigmoide Bindungscharakteristik, da hierdurch der Sauerstoff aus der Luft in großen Mengen gebunden und somit gespeichert und zugleich effektiv wieder diffusiv, gemäß dem FICKschen Gesetz, an die vitalen Zellschichten der Epidermis abgegeben werden kann. Wird zusätzlich Myoglobin eingesetzt, so hat dies den weiteren Vorteil, dass dessen Molekulargewicht viermal kleiner ist als das des Hämoglobins, so dass ein noch tieferes Eindringen des Sauerstofftransportmoleküls in die Haut möglich ist.

Die Bindung der Hämoglobine für Sauerstoff läßt sich durch zwei quantitative Größen hinreichend charakterisieren. Dies ist der sogenannte SauerstoffPartialdruck der Halbsättigung (p50) des betreffenden Hämoglobins mit Sauerstoff, welcher ein umgekehrtes Maß für die mittlere Affinität des Sauerstoffs zum Hämoglobin darstellt, und das ist der sogenannte HILLsche Index (n50), der ein Maß für die Sigmoidität der Sauerstoffbindungskurve des Hämoglobins darstellt. Dieser Index des menschlichen Hämoglobins im Blut beträgt unter physiologischen Bedingungen etwa 2,6 und sollte bei allen Bereitungen möglichst so groß erhalten bleiben. Der p50-Wert dagegen sollte optimiert werden.

Mit Hilfe von Effektoren der Sauerstoffbindung kann dies noch weiter mit der erfindungsgemässen Zubereitung verbessert werden. Es ist daher bevorzugt, dass bei der Herstellung der Lösung des Sauerstoffträgers bekannte natürliche Effektoren, wie z.B. 2,3.Diphosphoglycerat oder künstliche Effektoren wie Inositolhexaphosphat oder Mellitsäure, in 1-3facher, insbesondere etwa äquivalenten Mengen, bezogen auf Hämoglobin oder Hämoglobin/Myoglobin, hinzugesetzt werden (Barnikol et al. Funkt.Biol.Med. 2 (1983) 245-249). Natürliche Effektoren, welche nicht chemisch reagieren, d.h. konformativ an das Hämoglobin/Myoglobin gebunden sind ,sind beispielsweise in "Lehninger et al.," Prinzipien der Biochemie", Spektrum-Verlag, 1994 beschrieben.
Darüberhinaus kann Hämoglobin oder Myoglobin auch bevorzugt, auch zusätzlich zu den obengenannten Effektoren, chemisch modifiziert sein mit Effektoren, welche kovalent an das Hämoglobin gebunden werden. Hierzu gehören beispielsweise Pyridoxal-5-phosphat. Die Herstellung solcher modifizierter Hämoglobine ist in Kothe et al. Surgery 161 (1985), 55, 563-569 beschrieben. Alternativ kann auch 2-Nor-2-Formyl-Pyridoxal-5-phosphat (van der Plas et al; Transfusion 27 (1985)425-430) als Effektor verwendet werden. Weitere Zitate finden sich in: Rudolph A.S. et al, (Hsrg.): Red Blood Cell Substitutes: Basic Principles and Clinical Applications, Marcel Dekker, New York u.a. 1998; Tsuchida E. (Hrsg.): Blood Substitutes: Present and Future Perspektives, Elsevier Science, Amsterdam 1998; Chang T.M.S. (Autor bzw. Hrsg.): Blood Substitutes: Principles, Methods, Products and Clinical Trials, Volume 1 und Volume 2, Karger Landes, Basel, u.a. 1997 und 1998, vgl. auch EP 0 528 841, dort wird die Pyridoxylierung von Hämoglobin beschrieben. Kovalent bindende Effektoren können sowohl für Hämoglobin als auch für Myoglobin verwendet werden.

Mit Hilfe der erfindungsgemäßen Zubereitung werden überraschenderweise die Eigenschaften der molekular-dispersen Hämoglobine für eine möglichst effektive perkutane Sauerstoffdiffusion so optimiert, dass eine günstige Sauerstoffversorgung der Haut extern möglich wird. Darüberhinaus wird durch die erfindungsgemäße Zubereitung, worin Hämoglobin/Myoglobin in eine wasserbindende und tiefenwirksame gelartige Struktur insbesondere ein Gel eingebracht sind und das Wasser der gelartigen Struktur die oberen Hautschichten aufquillt, günstigerweise der Diffusionswiderstand für Sauerstoff verringert.

Solche, mit Hilfe des Mechanismus der erleichterten Diffusion, Sauerstoff-transportierende Hämoglobin/Myoglobin-haltigen gelartigen Zubereitungen sind nicht nur medizinisch von großem Interesse, sondern auch aus Sicht der Kosmetik. Denn der Alterungsprozeß der Haut ist auch entscheidend durch eine verringerte Sauerstoff-Verfügbarkeit für die vitale, hochaktive Zellschicht der Epidermis mitbedingt.

Daher eignen sich die erfindungsgemäßen Hämoglobin- oder Hämoglobin/Myoglobin-haltigen Zubereitungen insbesondere auch als Mittel zur Behandlung von altersbedingtem Sauerstoffmangelzuständen der Haut, neben der Behandlung der Sauerstoffmangelzustände allgemein oder durch dauergenerative und/oder strahlungs- sowie thermisch bedingte Hautveränderungen, sowohl präventiv als auch therapeutisch, insbesondere auch als gleichzeitige Co-Therapie mit intravasaler Anwendung künstlicher Sauerstoffträger.

Als Hämoglobin kann insbesondere Humanhämoglobin, Schweinehämoglobin oder Rinderhämoglobin eingesetzt werden. Die Art des Myoglobins ist ebenfalls wählbar, es kann von verschiedenen Tierarten gewonnen werden, wie z.B. vom Hund, vom Schaf, vom Pferd oder vom Wal. Die Hämoglobine können jeweils nativ oder bevorzugt wie geschildert mit einem Effektor wie oben beschrieben, versehen und/oder gegen Oxidation geschützt sein.Darüberhinaus kann die Zubereitung bevorzugt einen natürlichen, konformativ gebunden Effektor wie oben geschildert aufweisen.
Gegen Oxidation kann Hämoglobin wie auch Myoglobin beispielsweise karbonyliert, d.h. mit Kohlenmonoxid (CO) versehen und somit stabilisiert werden.

Wird das erfindungsgemäße Präparat in stabilisierter Form in die Haut eingbracht, wird Hämoglobin oder das Hämoglobin und Myoglobin mit reinem Sauerstoff durch kurzzeitiges, etwa halbstündiges Begasen von außen, jedoch ohne Überdruck, als Sauerstoff-Binder reaktiviert, d.h. der Stabilisator wird entfernt. Von da ab transportiert der gelöste künstliche Sauerstoffträger diffusiv verstärkt Sauerstoff auch aus der Luft, welche nur rund 20% Volumenprozent Sauerstoff enthält. Es ist somit bevorzugt, dass das eingesetzte Hämoglobin oder Myoglobin gegen Oxidation geschützt, also stabilisiert ist.

Alternativ kann das Sauerstoff-transportierende Hämoglobin/Myoglobin auch ohne Stabilisation (Oxidationsschutz) angewendet werden. Obgleich ein solches Präparat nicht so lange haltbar ist wie ein CO-stabilisiertes, hat es den Vorteil, direkt ohne vorherige Aktivierung mit reinem Sauerstoff wirken zu können.

Das nicht stabilisierte Produkt eignet sich daher eher für die häusliche Anwendung, während das stabilisierte Produkt insbesondere für die ambulante oder stationäre Ersttherapie zum Einsatz kommen kann.

Die oben genannten Hämoglobine/Myoglobine sind als solche bekannt und beispielsweise in "Prinzipien der Biochemie" von Lehninger, Nelson, Cox (Spektum-Verlag) beschrieben.

Die eingesetzten Hämoglobine und Myoglobine können alternativ auch insbesondere mit Polyalkylenoxiden kovalent verknüpft sein, wie in US 4 179 337, US 5 478 805, US 5 386 014, EP 0 206 448, EP 0 67 029 beschrieben. Dies dient der Gewebeverträglichkeit der Produkte.

Kovalente Anknüpfungen von Polyalkylenoxiden an Proteine, insbesondere auch an (unvernetztes) Hämoglobin, sind etliche bekannt und in der Literatur beschrieben (den Stand der Techhik beschreibt umfassend: Harris J. M. (Hrsg.): *Poly (Ethylene Glycol) Chemistry: Biotechnical and Biomedical Applications*, Plenum, New York u. a. 1992). Bei sehr vielen dieser Verfahren erfolgt die Anknüpfung des Polyalkylenoxids über eine molekulare Brücke (*"spacer*"), die beispielsweise ein bifunktioneller Verknüpfer schafft. Streng betrachtet wird in diesen Fällen also ein Verknüpfungsprodukt eines Polyalkylenoxids mit einem Verknüpfungsreagenz an das Protein geknüpft.
Zur kovalenten, Anknüpfung der Polyalkylenoxide (Polyalkylenglykole) werden bevorzugt solche Derivate der Polyalkylenoxide verwendet, die ein verknüpfendes Agens mit einer funktionellen Gruppe bereits kovalent gebunden enthalten, welche eine direkte chemische Reaktion mit Amino-, Alkohol-, oder Sulfhydryl-Gruppen der Hämoglobine unter Bildung kovalenter Anknüpfungen der Polyalkylenoxide ergeben - beispielsweise. Polyalkylenoxide mit reaktiven N-Hydroxysuccinimidylester-, Epoxid- (Glycidylether-), Aldehyd-, Isocyanat-, Vinylsulfon-, Jodazetamid-, Imidazolylformat-, Tresylatgruppen, u. a. Viele solche monofunktionell aktivierte Polyethylenglykole sind kommerziell erhältlich. Alternativ können nicht-aktive Polyalkylenoxide in jeder weiteren geeigneten Weise zunächst chemisch aktiviert oder, eventuell nach einer zusätzlich notwendigen Derivatisierung, durch chemische Verknüpfungsagenzien mit dem Hämoglobin verknüpft werden, beispielsweise mittels chemischer Reaktion mit Bromcyan, einem Karbodiimid wie beispielsweise 1-Ethyl-3-(3-Dimethylaminopropyl)karbodiimid oder N,N'-Dizyklohexylkarbodiimid, Cyanurchlorid (mit diesem aktivierte Polyethylenglykole, 4,6-Dichlor-s-triazin-Polyethylenglykole, sind ebenfalls kommerziell erhältlich), oder anderen, bekannten Verknüpfungsagenzien wie beispielsweise 2,2'-Dichlorbenzidin, p,p'-Difluor-m,m'-dinitrodiphenylsulfon, 2,4-Dichlornitrobenzol und weiteren (Überblick in: Harris J. M. (Hrsg.): *Poly (Ethylene Glycol) Chemistry: Biotechnical and Biomedical Applications,* Plenum, New York u. a. 1992).

Als Polyalkylenoxide eignen sich besonders Polyethylenglykole (Polyethylenoxide), Polypropylenglykole (Polypropylenoxide), sowie Kopolymere aus Ethylenglykol (Ethylenoxid) und Propylenglykol (Propylenoxid), insbesondere gewisse Derivate dieser.
Wie bereits erwähnt ist die Anbindung von Polyalkylenoxiden an Proteine (z. B.: Patent US 4,179,337 (1979): "Non-immunogenic Polypeptides"), speziell auch an Hämoglobine, namentlich auch an künstliche Sauerstoffträger auf der Basis modifizierter Hämoglobine, bekannt (Patentschriften US 5,478,805 (1995): "Fractionation of Polyalkylene Oxide-Conjugated Hemoglobin Solution", US 5,386,014 (1995): "Chemically Modified Hemoglobin as an Effective, Stable, Non-immunogenic Red Blood Cell Substitute", EP-A 0 206 448 (1986): "Hemoglobin Combined with a Poly (Alkylene Qxide)", EP-A 0 067 029 (1982): "Oxygen Carrier"). Der Inhalt dieser Schriften ist daher vorliegend inkorporiert. Die Anknüpfung von Polyalkylenoxiden an künstliche Sauerstoffträger auf der Basis modifizierter Hämoglobine wird nach der bekannten Literatur nur an unvernetztem Hämoglobin vorgenommen.

So beschreibt EPA 0 067 029 die Anknüpfung von Polyalkylenglykol, z.B. Polyethylen-/propylenglykol oder Copolymeren von Ethylenoxid/Propylenoxid oder einem Ether der genannten Glykole mit einem C₁-C₁₆-Alkohol, einem Ester aus den genannten Glykolen mit einer C₂-C₁₈-Carbonsäure (vorzugsweise Butyl/Monostearylester) und einem Amid aus Glykol und einem C₁-C₁₈-Amin (z.B. Propyl-Stearylamin). Als Verknüpfer werden z.B. N-Hydroxysuccinimid, N-Hydroxyphthalimide, p-Nitrophenol, Pentachlorphenol erwähnt. Analog können reaktive Derivate der genannten Polyalkylenglykol-Produkte eingesetzt werden.
Das Molekulargewicht der Polymeren (.z.B. Polyether) kann 300-20000, insbesondere 750-10000 sein. Molare Mengenverhältnisse und Reaktionstemperatur richten sich nach den jeweiligen beschriebenen und bekannten Bedingungen (vgl. Beispiele), z.B. 1-40-facher Überschuss Polyalkylenoxid/Derivat pH 7-10.
Auch können Hämoglobin/Myoglobin mit Effektoren hier - wie erwähnt - verbunden sein, wie z.B. Pyridoxal-5'-phosphat, Pyridoxal-5'-sulfat.

Die EPA 0 206 448 beschreibt ebenfalls die Anknüpfung von Polyalkylenoxiden wie obengenannt, welche eine Aminofunktion aufweisen und somit über eine Amidbindung mit Hämoglobin, verbunden sind. Das Molekül weist die Formel -CH₂-O-(CH₂)ₙ-CONH Hb (n > 1. insbesondere 1-10) auf. In den Beispielen 1-5 wird die Verknüpfung, z.B. mit derivatisiertem Polyethylenglykol, beschrieben, z.B. auch bei Verwendung von Pyridoxal-5'-phosphat-Hämoglobin.

Die US-Patente 5 312 808 und 5 478 805 beschreiben die Herstellung von Hämoglobin-enthaltenden Lösungen mit Polyalkylenoxid-konjugiertem Hämoglobin mit einem Molekulargewicht größer 85000 Dalton, vgl. insbesondere 'die Beispiele 1-4, worin die Reaktionsbedingungen angegeben sind.

US-A 5 234 903 offenbart (siehe Beispiele) ein mit einem Polyalkylenoxid (z.B. PEG) verknüpftes Hämoglobin, welches insbesondere auch über eine Carbamidbindung (Urethan) verknüpft sein kann. In den Beispielen I-V sind insbesondere molare Mengen und Reaktionsbedingungen für die Anknüpfung von Polyethylenglykol an Hämoglobin angegeben.

Gemäß der DE-OS 30 26 398 wird (nicht aktiviertes) Polyethylenglykol mit der 2-bis 5-fachen molaren Menge Bromcyan (pH-Wert 9-10) umgesetzt. Das restliche Bromcyan wird durch Gelfiltration, Dialyse etc. aus dem Reaktionsgemisch entfernt und das Produkt wird dann mit einer erforderlichen, z.B. 0,1- bis 0,002-fachen, Menge Hämoglobin (pH 7 bis 9) in wässriger Lösung umgesetzt. Alternativ wird Polyethylenglykol in Benzol gegeben und mit der 2- bis 5-fachen molaren Menge Cyansäurechlorid umgesetzt. Das Reaktionsprodukt, Polyethylenglykol-4,5-dichlors-triazin, wird mit der gewünschten Menge, z.B. 1 bis 0,002 mol, Hämoglobin in einer Pufferlösung umgesetzt.

Die vorstehend erläuterten Methoden lassen sich auch im Fall der anderen genannten Polymeren sowie auch auf Myoglobin anwenden.

Bevorzugt wird also monomeres Hämoglobin/Myoglobin, insbesondere desoxygeniert, in wässrigen Elektrolyten (z.B. NaHCO₃, NaCl, Na-Lactat oder Mischungen hiervon) mit einem Überschuss an Polyalkylenoxid, beispielsweise Polyethylen/propylenglykol (-oxid), Copolymerisate hiervon oder Derivate hiervon, insbesondere einem aktivierten Polyethylenglykol wie Methoxy-Polyethylenglykol-N-Hydroxysuccinimidylpropionat (mPEG-SPA) mit dem gewünschten Molekulargewicht wie beschrieben, vernetzt werden. Der Überschuss an Reaktand kann auf bekahnte Weise (Lysin) entfernt werden. Dabei kann vorzugsweise ein Effektor z.B. kovalent angeknüpft sein, wie geschildert, und/oder insbesondere auch nur ein konformativ wirksamer Effektor der obengenannten Art der Lösung später zugesetzt werden. Ein wie oben beschrieben hergestelltes Hämoglobin/Myoglobin kann z.B. chromatographisch (z.B. durch präparative Volumenausschluss-Chromatographie, wie z.B. an Sephadex G-25 Gel) durch Zentrifugation, Filtration oder Ultrafiltration gereinigt und nachfolgend in der beschriebenen Weise zum erfindungsgemässen Gel weiterverarbeitet werden, wobei die Reinigungsmethoden in den obengenannten Druckschriften ebenfalls beschrieben sind, vgl. auch Curling J.M.: Methods of Plasma Protein Fractionation, Academic Press, London, 1980, oder EP-A 0 854 151, EP-A 95 107 280. Gegebenenfalls erfolgt Stabilisierung durch Karbonylierung.

Alternativ wird nicht modifiziertes natives Hämoglobin und Myoglobin eingesetzt, welches insbesondere bevorzugt durch Karbonylierung gegen Oxidation geschützt sein kann, wobei die Sauerstoffträgerlösung einen nicht chemisch reaktiven Effektor, wie erwähnt insbesondere 2,3-Diphophoglycerat aufweist, in einer 1 bis 3 fachen, vorzugsweise äquivalenten Menge bezüglich des Hämoglobins/Hämoglobins/Myoglobins. Ferner kann auch mit Pyridoxal-Effektoren chemisch modifiziertes Hämoglobin wie von Kothe und van der Plas beschrieben eingesetzt werden. Dazu wird Hämoglobin mit den entsprechenden genannten Effektoren umgesetzt, gegebenenfakks karbonyliert. Der Lösung kann dann bevorzugt ein nicht chemich reaktiver Effektor zugesetzt werden.

Ganz bevorzugt wird erfindungsgemäss desoxygeniertes, gegebenenfalls karbonyliertes nicht modifizertes Human- oder insbesondere Schweinehämoglobin und entsprechendes desoxygeniertes, nicht modifiziertes Myoglobin vom Hund, oder Schaf, Pferd eingesetzt.

Die pharmazeutische Zubereitung lässt sich beispielhaft wie folgt herstellen:

Zunächst wird eine gelbildende Substanz, beispielsweise Hydrogel, bevorzugt, solche mit dermaler Tiefenwirkung, insbesondere bevorzugt vom Typ der anionischen Polyacrylate, (wie Carbopol ®) in aqua conservans gelöst. Aqua conservans kann aus der Apotheke bezogen oder gemäß NRF (Neues Rezept Formulatorium) S.6. selbst hergestellt werden, Zusammensetzung von aqua conservans siehe dort. Dazu verwendet man gereinigtes Wasser und fügt die Konservierungsmittel, insbesondere 0,02 bis 0,08 Propyl-4-Hydroxybenzoat, bevorzugt jedoch 0,025 sowie 0,07 bis 0,15 Masseteile Methyl-4-Hydroxybenzoat, bevorzugt jedoch 0,075 hinzu.
Zur Herstellung des streichfähigen Gels werden 0,1 bis 50, bevorzugt 1 bis 20g, einer Gelbildenden Substanz wie ein Hydrogel, insbesondere 1-5g Carbopol ®, z.B. bevorzugt 1-5gauf 1 Liter aqua conservans gegeben und gelöst.
Als Feuchthaltemittel, zum Weichmachen der Haut, setzt man zwischen 5% und 15% Masseanteile z.B. an Glycerol, Propylenglykol oder 70%ige Sorbitlösung gemäß DAB 9, bevorzugt 8 bis 12% zu. Alternativ kann man zwei oder drei der genannten Feuchthaltemittel in der angegebenen (Gesamt)Menge, bevorzugt zu gleichen Teilen, insbesondere mit einem Gesamtmasse-Anteil zwischen 8 und 12%, zusetzen.
Die Gelbildung erfolgt nach Zugabe von Reaktionsmitteln wie Basen, insbesondere Natronlauge (NaOH), zwischen 2 und 25 ml normaler NaOH, bevorzugt jedoch zwischen 6 und 12 ml 1 N NaOH.
Anschließend wird eine konzentrierte Lösung des Hämoglobins oder des Hämoglobins und Myoglobins, insbesondere des Human-, Schweine- oder Rinderhämoglobins bzw. Rind/Schaf-Pferd Myoglobins, welche nicht modifiziert, oder auch bevorzugt chemisch modifiziert oder mit einem nicht chemisch reaktiven Effektor versehen sind, in einem Konzentrationsbereich zwischen 150 und 450, bevorzugt zwischen 300 und 400 g/l, welche insbesondere bevorzugt nach Schütteln mit reinem Kohlenmonoxid (CO) vollständig karbonyliert sind, derart in die fertige Gel-Zusammensetzung homogen eingemischt, dass das Gel einen Hämoglobin/Myoglobin-Gehalt zwischen 0,1 und 20% (Massenanteile), bevorzugt zwischen 2 und 8% besitzt. Den Hämoglobinen kann, wie erwähnt, bis zu 50 Gewichtsprozent Myoglobin beigemischt sein.

Im Falle der Herstellung einer Zubereitung für die häusliche und selbständige Nach-Therapie unterbleibt die Karbonylierung des Hämöglobins/Myoglobins.

Vorteil dieser Art Sauerstoff-Therapie , nämlich mittels erleichterter Diffusion durch hämoglobinhaltige Externa und im Gegensatz zu einer Behandlung mit reinem Sauerstoff im Überdruck, ist, daß hier den betreffenden Zellen Sauerstoff in 'großer Menge in "niedergespannter" Form, d.h. physiologischer Form, zugeführt werden kann. Hochgespannter Sauerstoff, d.h. Sauerstoff unter hohem Partialdruck, ist radikalisch aggressiv und wirkt toxisch, wie man aus der Intensivmedizin seit langem weiß.

Die Erfindung wird anhand des nachfolgenden Beispieles näher erläutert.

### Anwendungsbeispiel

Herstellung eines Sauerstoff- transportierenden Gels, das Schweine-Hämoglobin enthält.

1,5 g Methyl-4-Hydroxy-Benzoat und 0,5 g Propyl-4-Hydroxy-Benzoat wurden in bidestilliertem Wasser gelöst und mit diesem auf 1 I aufgefüllt (: "DAC"). 5,0 g Carbopol® 940 werden mit 45 ml Glycerol und 45 ml 1,2 - Propandiol angerührt, der Mischung dann 850 ml DAC hinzugefügt und ferner 350 ml bidestilliertes Wasser sowie 38 ml 1 M NaOH zur Gel-Bildung. In das Gel ließen sich 280 ml einer Schweinehämoglobin-Lösung der Konzentration 280 g/l homogen einmischen. Das Hämoglobin war vorher zu 99% mit Kohlenmonoxid ligandiert worden.

## Patentansprüche

1. Extern applizierbares Präparat gelartiger Konsistenz, **dadurch gekennzeichnet, dass** das Präparat 5 bis 15 % Gew. Feuchthaltemittel, 0,15 bis 0,25 Gew. % Konservierungsmittel, eine Gel-bildende Substanz in einer Menge von 0,1-20 Gew. % und Hämoglobin oder Myoglobin und Hämoglobin in einer Konzentration von 0,1 bis 20 Gew. %, bezogen auf die Gesamtmenge molekular - dispers eingearbeitet, aufweist.

2. Präparat nach einem der Anspruch 1, **dadurch gekennzeichnet, dass** das Hämoglobin oder Myoglobin und Hämoglobin in ein Hydrogel eingearbeitet ist.

3. Präparat nach einem der Ansprüche 1 oder 2, **dadurch gekennzeichnet, dass** als Hämoglobin Human-, Schweine- oder Rinderhämoglobin und als Myoglobin solches vom Pferd, Hund oder Schaf eingearbeitet ist.

4. Präparat nach einem der Ansprüche 1 bis 3, **dadurch gekennzeichnet, dass** als Hämoglobin oder Myoglobin und Hämoglobin natives, chemisch modifiziertes und/oder gegen Oxidation geschütztes Hämoglobin oder Myoglobin und Hämoglobin eingearbeitet ist.

5. Präparat nach einem der Ansprüche 3 oder 4, **dadurch gekennzeichnet, dass** das Hämoglobin oder Myoglobin und Hämoglobin mit einem Polyalkylenoxid kovalent verknüpft und/oder mit einem natürlichen und/oder künstlichen Effektor kovalent und/oder konformativ versehen ist.

6. Präparat nach einem der Ansprüche 1 bis 5, **dadurch gekennzeichnet, dass** eine Lösung von Hämoglobin oder Hämoglobin und Myoglobin in Wasser, enthaltend Salze wie NaCl, KCI und NaHCO₃, insbesondere in physiologischen Mengen in mM: NaCl: 125; KCI: 4,5; NaHCO₃: 2,0 eingearbeitet ist.

7. Präparat nach einem der Ansprüche 1 bis 6, **dadurch gekennzeichnet, dass** Hämoglobin/ Myoglobin karbonyliert ist.

8. Verfahren zur Herstellung eines Präparates gemäß einem der Ansprüche 1 bis 7, **dadurch gekennzeichnet, dass** man eine Gel-bildende Substanz in Wasser einarbeitet, diesem gegebenenfalls Zusatzstoffe zusetzt, anschließend die Gelbildung durch Zugabe von Reaktionsmitteln initiiert und sodann eine Hämoglobinlösung oder Myoglobinlösung und Hämoglobinlösung eines Konzentrationsbereiches von 150 bis 450 g/l so zusetzt, dass das Gel 0,1 bis 20 Gew. % an Hämoglobin oder Hämoglobin und Myoglobin aufweist.

9. Verfahren nach Anspruch 8, **dadurch gekennzeichnet, dass** als Gel-bildende Substanz ein Hydrogel und als Reaktionsmittel NaOH zugesetzt werden.

10. Verfahren nach einem der Ansprüche 8 oder 9, **dadurch gekennzeichnet, dass** als Zusatz Konservierungsmittel, ausgewählt aus Methyl-4-hydroxybenzoat und Propyl-4-hydroxybenzoat oder Mischungen hiervon, Sorbit, Propylenglycol und Glycerol oder Mischungen hiervon als Feuchthaltemittel zugesetzt werden.

11. Verfahren nach einem der Ansprüche 8 bis 10, **dadurch gekennzeichnet, dass** als Hämoglobin ein wie in Anspruch 3 bis 4 beschriebenes Hämoglobin /Myoglobin zugesetzt wird.

12. Verwendung eines Präparates gemäss einem der Ansprüche 1 bis 7 oder hergestellt gemäss einem der Ansprüche 8 bis 11 zur Herstellung eines Mittels zur externen Behandlung/ Prävention von Sauerstoffmangelzuständen der Haut.

13. Verwendung gemäss Anspruch 12, **dadurch gekennzeichnet, dass** der Sauerstoffmangel durch degenerative und/oder strahlungs- sowie thermischbedingte Hautveränderungen hervorgerufen ist.

14. Verwendung eines Hämoglobin- haltigen Präparates gemäss einem der Ansprüche 1 bis 7 oder hergestellt gemäss einem der Ansprüche 8 bis 11 zur Herstellung eines Mittels zur Behandlung/ Prävention von altersbedingten Sauerstoffmangelzuständen der Haut.

15. Verwendung gemäss einem der Ansprüche 12 bis 14, **dadurch gekennzeichnet, dass** ein stabilisiertes Gel-Präparat angewendet wird und zur Aktivierung des Hämoglobins/Myoglobins - Hämoglobins als Sauerstofftransporter eine Begasung der Haut mit reinem Sauerstoff vorgenommen wird.

16. Verwendung gemäss einem der Ansprüche 12 bis 14, **dadurch gekennzeichnet, dass** ein nicht stabilisiertes Hämoglobin/Hämoglobin oder Myoglobin für die häusliche Therapie eingesetzt wird.

## Claims

1. Externally applicable preparation of gel-like consistency, **characterised in that** the preparation contains 5 to 15% by weight of humectant, 0.15 to 0.25% by weight of preservative, a gel-forming substance in an amount of 0.1 to 20% by weight and haemoglobin or myoglobin and haemoglobin in a concentration of 0.1 to 20% by weight, based on the total amount incorporated, molecularly dispersed.

2. Preparation according to claim 1, **characterised in that** the haemoglobin or myoglobin and haemoglobin are incorporated in a hydrogel.

3. Preparation according to either claim 1 or claim 2, **characterised in that** human, pig or bovine haemoglobin is incorporated as haemoglobin, and horse, dog or sheep myoglobin is incorporated as myoglobin.

4. Preparation according to any one of claims 1 to 3, **characterised in that** native or chemically modified haemoglobin or myoglobin and haemoglobin and/or haemoglobin or myoglobin and haemoglobin, protected against oxidation, is/are incorporated as haemoglobin or myoglobin and haemoglobin.

5. Preparation according to either claim 3 or claim 4, **characterised in that** the haemoglobin or myoglobin and haemoglobin is linked covalently with a polyalkylene oxide and/or provided covalently and/or conformatively with a natural and/or an artificial effector.

6. Preparation according to any one of claims 1 to 5, **characterised in that** a solution of haemoglobin or myoglobiu and haemoglobin is incorporated in water, containing salts such as NaCl, KCl and NaHCO₃, in particular in physiological amounts in mM: NaCl:125; KCl:4.5; NaHCO₃:2.0.

7. Preparation according to any one of claims 1 to 6, **characterised in that** the haemoglobin/myoglobin is carbonylated.

8. Method for preparing a preparation according to any one of claims 1 to 7, **characterised in that** a gel-forming substance is incorporated in water, additives optionally are added, subsequently the gel formation is initiated by the addition of reactants and then a haemoglobin solution or a myoglobin solution and haemoglobin solution, having a concentration ranging from 150 to 450 g/l, is added in such a manner, that the gel contains 0.1 to 20% by weight of haemoglobin or haemoglobin and myoglobin.

9. Method according to claim 8, **characterised in that** a hydrogel is added as gel-forming substance and NaOH as reactant.

10. Method according to either claim 8 or claim 9, **characterised in that**, as additive, preservatives, selected from methyl 4-hydroxybenzoate and propyl 4-hydroxybenzoate or mixtures hereof, and humectants, selected from sorbitol, propylene glycol and glycerol or mixtures hereof, are added.

11. Method according to any one of claims 8 to 10, **characterised in that** a haemoglobin/myoglobin as described in claim 3 and claim 4 is added as the haemoglobin.

12. Use of a preparation according to any one of claims 1 to 7 or prepared according to any one of claims 8 to 11 for preparing an agent for the external treatment/prevention of oxygen deficiency conditions in the skin.

13. Use according to claim 12, **characterised in that** the oxygen deficiency is caused by degenerative and/or radiation-induced or thermally induced skin changes.

14. Use of a haemoglobin-containing preparation according to any one of claims 1 to 7 or prepared according to any one of claims 8 to 11 for preparation of an agent for the treatment/ prevention of age-related conditions of oxygen deficiency in the skin.

15. Use according to any one of claims 12 to 14, **characterised in that** a stabilised gel preparation is used and, in order to activate the haemoglobin/myoglobin-haemoglobin as oxygen carrier, the skin is gassed with pure oxygen.

16. Use according to any one of claims 12 to 14, **characterised in that** a haemoglobin or haemoglobin and myoglobin, which has not been stabilised, is used for domestic therapy.

## Revendications

1. Préparation applicable extérieurement de consistance du genre gel, **caractérisée en ce que** la préparation présente 5 à 15 % en poids d'agent de conservation de l'hydratation, 0,15 à 0,25 % en poids d'agent conservateur, une substance formant un gel en une quantité de 0,1 - 20 % en poids et de l'hémoglobine ou de la myoglobine et de l'hémoglobine en une concentration de 0,1 à 20 % en poids rapporté à la quantité totale, incorporées de manière dispersée-moléculaire.

2. Préparation selon la revendication 1, **caractérisée en ce que** l'hémoglobine ou la myoglobine et l'hémoglobine sont incorporées dans un hydrogel.

3. Préparation selon l'une des revendications 1 ou 2, **caractérisée en ce qu'**on incorpore comme hémoglobine de l'hémoglobine humaine, de porc ou de boeuf et comme myoglobine celle de cheval, de chien ou de mouton.

4. Préparation selon l'une des revendications 1 à 3, **caractérisée en ce qu'**on incorpore comme hémoglobine ou myoglobine et hémoglobine de l'hémoglobine ou de la myoglobine et de l'hémoglobine natives, modifiées chimiquement et/ou protégées contre l'oxydation.

5. Préparation selon l'une des revendications 3 ou 4, **caractérisée en ce que** l'hémoglobine ou la myoglobine et l'hémoglobine sont combinées par covalence avec un polyoxyalkylène et/ou sont prévues covalentes et/ou conformatives avec un effecteur naturel et/ou artificiel.

6. Préparation selon l'une des revendications 1 à 5, **caractérisée en ce qu'**on incorpore une solution d'hémoglobine ou d'hémoglobine et de myoglobine dans l'eau, contenant des sels comme NaCl, KCI et NaHCO₃ ; en particulier en quantités physiologiques en mM : NaCl : 125 ; KCL : 4,5 ; NaHCO₃ : 2,0.

7. Préparation selon l'une des revendications 1 à 6, **caractérisée en ce que** l'hémoglobine / myoglobine est carbonylée.

8. Procédé de préparation d'une préparation selon l'une des revendications 1 à 7, **caractérisé en ce qu'**on incorpore une substance formant un gel dans l'eau, celle-ci étant éventuellement additionnée d'additifs, ensuite on initie la formation de gel par addition de réactifs et ensuite on ajoute une solution d'hémoglobine ou de myoglobine et d'hémoglobine dans une plage de concentration de 150 à 450 g/l de manière que le gel présente 0,1 à 20 % en poids d'hémoglobine ou d'hémoglobine et de myoglobine.

9. Procédé selon la revendication 8, **caractérisé en ce qu'**on ajoute comme substance formant un gel un hydrogel et comme agent de réaction NaOH.

10. Procédé selon l'une des revendications 8 ou 9, **caractérisé en ce qu'**on ajoute comme additif un agent de conservation choisi parmi le 4-hydroxybenzoate de méthyle et le 4-hydrocybenzoate de propyle ou leurs mélanges, comme agent de conservation de l'hydratation le sorbitol, le propylèneglycol et le glycérol ou leurs mélanges.

11. Procédé selon l'une des revendications 8 à 10, **caractérisé en ce qu'**on ajoute comme hémoglobine une hémoglobine / myoglobine décrite dans les revendications 3 à 4.

12. Utilisation d'une préparation selon l'une des revendications 1 à 7 ou préparée selon l'une des revendications 8 à 11 pour la préparation d'un moyen pour le traitement / la prévention externe d'états de manque d'oxygène de la peau.

13. Utilisation selon la revendication 12, **caractérisée en ce que** le manque d'oxygène est provoqué par des altérations cutanées dégénératives et/ou occasionnées par le rayonnement ainsi que par la chaleur.

14. Utilisation d'une préparation contenant de l'hémoglobine selon l'une des revendications 1 à 7 ou préparée selon l'une des revendications 8 à 11 pour la préparation d'un moyen pour le traitement / la prévention d'états de manque d'oxygène consécutifs au vieillissement de la peau.

15. Utilisation selon l'une des revendications 12 à 14, **caractérisée en ce qu'**on utilise une préparation de gel stabilisée et que pour activer l'hémoglobine / la myoglobine - l'hémoglobine comme transporteur d'oxygène on effectue un gazage de la peau avec de l'oxygène pur.

16. Utilisation selon l'une des revendications 12 à 14, **caractérisée en ce qu'**on utilise une hémoglobine / hémoglobine ou myoglobine non stabilisée pour la thérapie de la peau.
